# NEW EUROPEAN PATENT SPECIFICATION

(11) **EP 0 168 506 B2**
(45) Date of publication and mention of the opposition decision: **07.01.1998**
(45) Mention of the grant of the patent: 19.12.1990
(21) Application number: 84107985.8
(22) Date of filing: 07.07.1984
(51) Int. Cl.: A61K 39/395, A61K 38/16, C07K 16/00

(54) **Process for preparing gamma globulin suitable for intravenous administration**
Verfahren zur Herstellung von gamma-Globulin zur intravenösen Anwendung
Procédé de préparation de gamma-globuline pour l'injection intraveineuse

(43) Date of publication of application: 22.01.1986
(73) Proprietor: Armour Pharma GmbH, 35002 Marburg (DE)
(72) Inventor: Fiechtl, Jürgen-Dietrich Friedrich, Dr., D-3430 Witzenhausen 4 - Ziegenhagen (DE); Kerner, Bernhard, Dr., D-4400 Münster (DE); Holzapfel, Jürgen, D-3440 Eschwege (DE); Puschmann, Martin, Dr., D-3446 Meinhard 1 (DE); Kimura, Tokusuke, Shinjuku-ku Tokyo 162 (JP); Kurosu, Fumio, Hasuda-Shi Saitama Prefecture 349-01 (JP)
(74) Representative: Werner, Hans-Karsten, Dr.Dipl.-Chem.

(56) References cited:
- EP-A- 0 078 331
- DE-A- 2 751 717
- US-A- 4 126 605

## Description

### Field of the invention

This invention reales to a product containing gamma globulin suitable for intravenous administration and a process for producing the product.

### Background of the invention

USP 4,126,605 (Schneider) describes a process for obtaining a product suitable for intravenous administration from Cohn Fraction II gamma globulin. Cohn Fraction II is dissolved in a buffered aqueous solution at pH 6.7. The solution contains hydroxyethyl starch. After filtering the solution, polyethylene glycol is added to a concentration of 10%. After removal of the precipitate, additional polyethylene glycol is added to the solution to a concentration of 20%. The precipitate which results is improved unmodified gamma globulin suitable for intravenous use.

USP 4,165,370 (Coval) describes a process for obtaining a product suitable for intravenous administration from Cohn fraction II gamma globulin. Cohn fraction II is dissolved in solution having an acid pH of 4.8 to 6.5 and a low ionic strength, i.e., having a conductance of about 300-10⁻⁶cm⁻¹ohm⁻¹. After filtering the solution, polyethylene glycol, molecular weight 4,000, is added first to a 4% concentrate, then 5% concentrate. After the solution is centrifuged and any precipitate is removed, additional polyethylene glycol is added to the solution to a 12% concentration. The resulting precipitate is an immunologically active unmodified gamma globulin suitable for intravenous use.

In the above mentioned processes, the yields of pure gamma globulin are about 30% of the gamma globulin of the Cohn Fraction II. Modifications have been made to increase yields, but the increased yield has generally been at the expense of purity. An increased yield at constant quality of the product has not yet been possible.

It is an object of the present invention to isolate gamma globulin of high purity, suitable for intravenous use, in high yield.

### Summary of the invention

An improved process for preparing gamma globulin suitable for intravenous administration comprising
(a) dissolving gamma globulin precipitated from blood or blood products in a solution;
(b) separating non-dissolved precipitate from solution;
(c) adding polyethylene glycol to the separated solution;
(d) separating precipitate from the polyethylene glycol solution;
(e) increasing the polyethylene glycol concentration in the solution;
(f) separating precipitated purified gamma globulin from the higher concentration polyethylene glycol solution;
(g) dissolving the purified gamma globulin in a solution suitable for intravenous administration wherein the improvement comprises
   (1) the solution in which the gamma globulin is dissolved is at a neutral pH;
   (2) in the first polyethylene glycol addition step, adding the polyethylene glycol to a concentration of 4.0―5.5% by weight;
   (3) in the second polyethylene glycol addition step, increasing the polyethylene glycol concentration to at least 9%, but not more than 16% by weight;
   (4) adding a citrate buffer to the solution just prior to adding the polyethylene glycol in one of the polyethylene glycol addition steps.

### Detailed description of the invention

### (a) Dissolving gamma globulin precipitated from blood products in solution

The gamma globulin useful as the starting material for the product and process of the current invention is well known in the art.

A particular process for the precipitation and isolation of gamma globulin from blood is known by the name "Cohn-Method" (Cohn et al., J. Amer. Chem. Soc., Vol. 68, pp. 459―475 and Vol. 72, pp. 465―474) or "Cohn Fraction II".

This gamma globulin preparation, unsuitable for intravenous use, is dissolved in an aqueous solution at a neutral pH.

The aqueous solution has a low ionic strength. The low ion concentration can be derived from salt present in the starting gamma globulin preparation or can be due to added buffer. All physiologically tolerated salts are suitable as buffers. These include phosphate, citrate and trihydroxy-ethyl-amino-methane.

The ionic concentration can be within the range of 0.001―0.015 mol/l. If buffer is added, it is preferred that the range be 0.01―0.015 mo/l.

The pH of the solution can be adjusted to 7.0±0.1 by addition of a suitable acid or base, for example, citric acid, sodium citrate or, if needed, sodium hydroxide.

It has been found that the higher the ionic concentration, the lower the temperature of the solution should be. If no additional buffer is used, the temperature of the solution may be room temperature. If the ionic concentrate is between .01―.015 mol/l, the temperature should be between 5―15°C.

The gamma globulin is dissolved in the solution in a concentration of 1―7% by weight. Preferably, the concentrate is 3.1―4.9%.

Also present in the solution may be "hydrocolloid" such as hydroxyethyl starch, dextrose, albumin, polyalcohol and polyvinyl pyrrolidone as disclosed in USP 4,126,605.

### (b) Separating non-dissolved precipitate from solution

After the gamma globulin has been dissolved, the insoluble impurities are removed from the solution by, for example, decantation, filtration, or centrifugation.

### (c) Adding polyethylene glycol to the separated solution

To the resultant supernatant is added polyethylene glycol (PEG) having a molecular weight between 2000―6000. Preferably, the PEG will have a molecular weight average of 4000.

The PEG may be added to the supernatant in bulk, as a powder or as solution having PEG dissolved therein.

PEG is added at room temperature to the separated solution to a concentration of 3―6% by weight, preferably 4.0―5.5% by weight.

It is important to add a citrate buffer to the gamma globulin solution just prior to adding PEG in one of PEG addition steps. The buffers useful are listed above. The ionic concentration of the solution after the addition of buffer should be 0.025―0.25 mol/l.

### (d) Separating precipitate from PEG solution

After the PEG is added to the 3―6% concentration, a precipitate is formed. The precipitate is removed by decantation, filtration or centrifugation.

### (e) Increasing the polyethylene glycol concentration

The concentration of PEG in the solution is increased to 9―16% by weight by the addition of PEG. The temperature of the solution at this step may be reduced to 0―10°C, however, it may remain at room temperature.

### (f) Separating precipitated purified gamma globulin

The purified immunoglobulin which precipitates after increasing the concentration of PEG is then separated by means of gentle separation procedures, for example, decantation, filtration or centrifugation. Preferably, the separation is by means of centrifugation.

The obtained purified gamma globulin is native, has low ACA and is suitable for use in products for intravenous administration.

### (g) Dissolving the purified gamma globulin in a solution suitable for intravenous administration

The purified gamma globulin is preferably dissolved in aqueous solution at a concentration of 2―10%, preferably about 5%. The solutions may also contain buffer, e.g., citrate and/or phosphate, sugar, e.g. glucose, maltose, sucrose, and an isotonicity agent, e.g. NaCl.

Preferred are solutions containing 2―3% by weight glucose and 5―50 mmol/l sodium citrate with a pH of 7.

In the process of the current invention yields are obtained of 70% or more of high quality product based on the amount of gamma globulin in the starting material. The average anti-complementary activity (ACA) of the product is approximately 10 CH₅₀ u/ml or less (protein concentrate of 5%).

### Example 1

Cohn-Fraction-II-Powder is dissolved in a 0.01 molar phosphate-citrate-buffer (7.00 pH, 10°C) with careful stirring to a protein concentration of 3.5%. Hydroxyethyl starch is present in a concentration of .5%.

The precipitate formed is removed and the solution is clarified by layer filtrations in one filtration step. Then the protein concentration is adjusted to 2.5% and the phosphate-citrate concentration is adjusted to 0.12 molar and the pH is adjusted to 7.0±0.1 by addition of a 0.5 molar phosphate-citrate-buffer (molarity related to content of phosphate/citrate). After the solution is heated to a temperature of 20°C, solid polyethylene glycol (PEG 4000 molecular weight 4000) to a concentration of 5.5% is added under careful stirring and completely dissolved.

Then the supernatant is decantated from the precipitate formed and is clarified by layer filtration.

The clarified supernatant is cooled to a temperature of 10°C and then diluted, with stirring, with a 50% solution of PEG 4000 in a 0.03 molar phosphate-citrate-buffer at 10°C to a PEG 4000 concentration of 14%. The precipitate formed (paste) is collected by continuous centrifugation.

The paste is dissolved in a 20 mmolar sodium-citrate-solution (pH=7.0±0.1) and 2.5% glucose is added. The so-prepared solution shows the following characteristic values:
- Protein:: 5.4%
- pH:: 7.00±0.05
- Glucose:: 2.5%±0.5%
- Osmolarity:: 300―330 mosmol/l
- ACA:: 10 U/ml
- HPLC: dimers+monomers:: 99%

The solution is sterile filtered and placed into vials and, optionally, lyophilized.

### Example 2

Cohn-Fraction-II-Powder is dissolved, at room temperature and pH of 7, in water for injection with careful stirring to a protein concentration of 5%. Hydroxyethyl starch is present in a concentration of .5%.

The precipitate formed is removed and the solution is clarified by layer filtration within one filtration step. Polyethylene glycol (PEG 4000), as a 40% solution, is added, under careful stirring, to a concentration of 4.0%.

The precipitate is removed by depth filtration.

To the clear supernatant .3M phosphate buffer is added to a concentration of 10% by volume and the PEG 4000 concentration is increased 10.4%.

The precipitate formed (paste) is collected by continuous centrifugation.

The paste is dissolved in a 10 mM citrate/10 mM phosphate buffer (pH 7.0±0.1) additionally containing 0.9% NaCl and 2.5 glucose.

The redissolved solution had the following characteristics:
- Protein:: 5.4%
- pH:: 7.00±0.05
- Glucose:: 2.5%±0.5%
- ACA:: 10 U/ml
- HPLC: dimers+monomers:: 99%

The solution is sterile filtered, filled into bottles, and, optionally freeze-dried.

The yield of immunoglobulin based on percentage of immunoglobulin in Cohn fraction II is excess of 70%.

## Claims

1. An improved process for preparing gamma globulin suitable for intravenous administration comprising
(a) dissolving gamma globulin precipitated from blood or blood products in a solution;
(b) separating non-dissolved precipitate from the solution;
(c) adding polyethylene glycol to the separated solution;
(d) separating precipitate from the polyethylene glycol solution;
(e) increasing the polyethylene glycol concentration in the solution;
(f) separating precipitated purified gamma globulin from the higher concentrated polyethylene glycol solution;
(g) dissolving the purified gamma globulin in a solution suitable for intravenous administration wherein the improvement comprises
(1) the gamma globulin precipitated from blood or blood products is dissolved in a solution having a neutral pH;
(2) in the first polyethylene glycol addition step, adding the polyethylene glycol to a concentration of 4.0―5.5% by weight;
(3) in the second polyethylene glycol addition step, increasing the polyethylene glycol concentration to at least 9%, but not more than 16% by weight;
(4) adding a citrate buffer to the solution just prior to adding the polyethylene glycol in one of the polyethylene glycol addition steps.

2. The process of claim 1 wherein the polyethylene glycol has a molecular weight average of about 4000.

3. A process of claim 1 further comprising the improvement of in step (a) dissolving the gamma globulin in a solution having an ion concentration of 0.001 to 0.05 mol/l at a temperature not exceeding 20°C.

4. A process of claim 1 wherein hydroxyethyl starch is added to the solution of step a.

5. A process of claim 1 wherein in step a) the gamma globulin is dissolved in a solution having an ion concentration in the range of 0.010―0.015 mol/l and at a temperature not exceeding 10°C.

6. A process of claim 1 wherein in step a) the gamma globulin is dissolved in an aqueous solution without added electrolyte and at a temperature of 15―30°C.

7. A process of claim 4 wherein the buffer addition is just prior to the first polyethylene glycol addition and it increases the ion concentration of the solution to 0.06―0.25 ml/l.

8. A process of claim 5 wherein the buffer addition is just prior to the second polyethylene glycol addition.

9. A process of claim 7 wherein the buffer is a 1/10 volume of 0.3 M phosphate buffer.

10. A process for preparing gamma globulin suitable for intravenous administration comprising
(a) dissolving 3.0―7.5% by weight Cohn Fraction II powder in a neutral pH aqueous solution containing .35―1% by weight hydroxy ethyl starch;
(b) separating any precipitate from solution;
(c) adding polyethylene glycol having a molecular weight of about 4000 to the solution to a concentration of 4.0―5.0%;
(d) separating the precipitate from the solution, then adding a citrate phosphate buffer to the solution to a concentrate of .02―.04 M;
(e) adding polyethylene glycol having a molecular weight of about 4000 to the solution to a concentration of 9.5―11.0%;
(f) separating the purified gamma globulin from the solution;
(g) dissolving the purified gamma globulin in a solution suitable for IV administration.

11. A product suitable for intravenous administration made by the process of claim 1.

12. A product suitable for intravenous administration made by the process of claim 9.

## Patentansprüche

1. Verbessertes Verfahren zur Herstellung von gamma-Globulin, das zur intravenösen Verabreichung geeignet ist, umfassend
(a) das Auflösen von gamma-Globulin, das aus Blut oder Blut-Erzeugnissen ausgefällt ist, in einer Lösung;
(b) das Abtrennen des nicht aufgelösten Niederschlags von der Lösung;
(c) das Hinzufügen von Polyethylenglycol zu der abgetrennten Lösung;
(d) das Abtrennen eines Niederschlags aus der Polyethylenglycol-Lösung;
(e) das Erhöhen der Polyethylenglycol-Konzentration in der Lösung;
(f) das Abtrennen des ausgefällten gereinigten gamma-Globulins aus der höher konzentrierten Polyethylenglycol-Lösung;
(g) das Auflösen des gereinigten gamma-Globulins in einer zur intravenösen Verabreichung geeigneten Lösung, worin die Verbesserung umfaßt, daß
(1) das aus dem Blut oder den Blut-Erzeugnissen ausgefällte gamma-Globulin in einer Lösung mit einem neutralen pH-Wert aufgelöst wird;
(2) in dem ersten Schritt des Hinzufügens von Polyethylenglycol das Polyethylenglycol bis zu einer Konzentration von 4,0 bis 5,5 Gew.-% zugegeben wird;
(3) in dem zweiten Schritt des Hinzufügens von Polyethylenglycol das Polyethylenglycol bis zu einer Konzentration von wenigstens 9 Gew.-%, jedoch nicht mehr als 16 Gew.-%, zugegeben wird;
(4) unmittelbar vor der Zugabe des Polyethylenglycols in einem der Schritte des Hinzufügens von Polyethylenglycol ein Citrat-Puffer zu der Lösung hinzugefügt wird.

2. Verfahren nach Anspruch 1, worin das Polyethylenglycol einen Mittelwert des Molekulargewichts von etwa 4 000 hat.

3. Verfahren nach Anspruch 1, weiterhin umfassend die Verbesserung, daß in Schritt (a) das gamma-Globulin in einer Lösung mit einer Ionen-Konzentration von 0,001 bis 0,05 mol/l bei einer Temperatur, die 20°C nicht überschreitet, aufgelöst wird.

4. Verfahren nach Anspruch 1, worin der Lösung von Schritt (a) Hydroxyethylstärke zugesetzt wird.

5. Verfahren nach Anspruch 1, worin in Schritt (a) das gamma-Globulin in einer Lösung mit einer Ionen-Konzentration im Bereich von 0,010 bis 0,015 mol/l und bei einer Temperatur, die 10°C nicht überschreitet, aufgelöst wird.

6. Verfahren nach Anspruch 1, worin in Schritt (a) das gamma-Globulin in einer wäßrigen Lösung ohne zugesetzten Elektrolyten und bei einer Temperatur von 15°C bis 30°C aufgelöst wird.

7. Verfahren nach Anspruch 4, worin die Zugabe des Puffers unmittelbar vor der ersten Zugabe von Polyethylenglycol vorgenommen wird und die Ionen-Konzentration der Lösung auf 0,06 bis 0,25 mol/l erhöht.

8. Verfahren nach Anspruch 5, worin der Puffer unmittelbar vor der zweiten Zugabe von Polyethylenglycol zugegeben wird.

9. Verfahren nach Anspruch 7, worin der Puffer 1/10 Volumen 0,3 M Phosphat-Puffer ist.

10. Verfahren zur Herstellung von gamma-Globulin, das zur intravenösen Verabreichung geeignet ist, umfassend
(a) das Auflösen von 3,0 bis 7,5 Gew.-% Pulver der Cohn-Fraktion II in einer wäßrigen Lösung von neutralem pH-Wert, die 0,35 bis 1 Gew.-% Hydroxyethylstärke enthält;
(b) das Abtrennen von etwaigem nicht aufgelösten Niederschlags aus der Lösung;
(c) das Hinzufügen von Polyethylenglycol mit einem Molekulargewicht von etwa 4 000 zu der abgetrennten Lösung bis zu einer Konzentration von 4,0 bis 5,0%;
(d) das Abtrennen eines Niederschlags aus der Lösung und dann das Hinzufügen eines Citrat-Phosphat-Puffers zu der Lösung bis zu einer Konzentration von 0,02 bis 0,04 M;
(e) das Hinzufügen von Polyethylenglycol mit einem Molekulargewicht von etwa 4 000 zu der Lösung bis zu einer Konzentration von 9,5 bis 11,0%;
(f) das Abtrennen des gereinigten gamma-Globulins aus der Lösung;
(g) das Auflösen des gereinigten gamma-Globulins in einer zur i.v.-Verabreichung geeigneten Lösung.

11. Zur intravenösen Verabreichung geeignetes Produkt, hergestellt mittels des Verfahrens nach Anspruch 1.

12. Zur intravenösen Verabreichung geeignetes Produkt, hergestellt mittels des Verfahrens nach Anspruch 9.

## Revendications

1. Un procédé perfectionné pour la préparation de gamma-globuline convenant à l'administration intraveineuse consistant à
(a) dissoudre dans une solution de la gamma-globuline précipitée à partir de sang ou de produits du sang;
(b) séparer le précipité non dissous de la solution;
(c) ajouter du polyéthylène-glycol à la solution séparée;
(d) séparer le précipité de la solution de polyéthylène-glycol;
(e) augmenter la concentration de polyéthyléne-glycol dans la solution;
(f) séparer la gamma-globuline purifiée précipitée à partir de la solution de polyéthylèneglycol plus fortement concentrée;
(g) dissoudre la gamma-globuline purifiée dans une solution convenant à l'administration intraveineuse, dans lequel le perfectionnement consist à
(1) dissoudre dans une solution ayant un pH neutre la gamma-globuline précipitée à partir de sang ou de produits du sang;
(2) dans la première étape d'addition de polyéthylène-glycol, ajouter le polyéthylène-glycol jusqu'à une concentration de 4,0 à 5,5% en poids;
(3) dans la seconde étape d'addition de polyéthylène-glycol, augmenter la concentration de polyéthylène-glycol jusqu'à au moins 9%, mais au plus 16% en poids;
(4) ajouter un tampon au citrate à la solution juste avant d'ajouter I pglyéthylène-glycol dans l'une des étapes d'addition de polyéthylène-glycol.

2. Le procédé de la revendication 1, dans lequel le polyéthylène-glycol a un poids moléculaire moyen d'environ 4000.

3. Un procédé de la revendication 1, comprenant de plus le perfectionnement consistant, dans l'étape (a), à dissoudre la gamma-globuline dans une solution ayant une concentration ionique de 0,001 à 0,05 mole/l à une température ne dépassant pas 20°C.

4. Un procédé de la revendication 1, dans lequel de l'amidon hydroxyéthylé est ajouté à la solution de l'étape (a).

5. Un procédé de la revendication 1, dans lequel, dans l'étape (a), la gamma-globuline est dissoute dans une solution ayant une concentration ionique comprise dans l'intervalle de 0,010 à 0,015 mole/l et à une température ne dépassant pas 10°C.

6. Un procédé de la revendication 1, dans lequel, dans l'étape (a), la gamma-globuline est dissoute dans une solution aqueuse sans électrolyte ajouté et à une température de 15 à 30°C.

7. Un procédé de la revendication 4, dans lequel l'addition de tampon est effectuée juste avant la première addition de polyéthylène-glycol et élève la concentration ionique de la solution entre 0,06 et 0,25 ml/l.

8. Un procédé de la revendication 5, dans lequel l'addition de tampon est effectuée juste avant la seconde addition de polyéthylène-glycol.

9. Un procédé de la revendication 7, dans lequel le tampon consiste en 1/10 de volume de tampon au phosphate 0,3 M.

10. Un procédé pour la préparation de gamma-globuline convenant à l'administration intraveineuse, consistant à
(a) dissoudre 3,0 à 7,5% en poids de poudre de Fraction II de Cohn dans une solution aqueuse à pH neutre contenant 0,35 à 1% en poids d'amidon hydroxyéthylé;
(b) séparer tout précipité de la solution;
(c) ajouter du polyéthylène-glycol ayant un poids moléculaire d'environ 4000 à la solution jusqu'à une concentration de 4,0 à 5,0%;
(d) séparer le précipité de la solution, puis ajouter un tampon citrate/phosphate à la solution jusqu'à une concentration 0,02 à 0,04 M;
(e) ajouter du polyéthylène-glycol ayant un poids moléculaire d'environ 4000 à la solution jusqu'à une concentration de 9,5 à 11,0%;
(f) séparer la gamma-globuline purifiée de la solution;
(g) dissoudre la gamma-globuline purifiée dans une solution convenant à l'administration intraveineuse.

11. Un produit convenant à l'administration intraveineuse, préparé par le procédé de la revendication 1.

12. Un produit convenant à l'administration intraveineuse, préparé par le procédé de la revendication 9.
